# EUROPEAN PATENT SPECIFICATION

(11) **EP 0 370 470 B1**
(45) Date of publication and mention of the grant of the patent: **03.11.1993**
(21) Application number: 89121530.3
(22) Date of filing: 21.11.1989
(51) Int. Cl.: A61K 7/021, A61K 7/13

(54) **Fluorescent cosmetic compositions**
Fluoreszierende kosmetische Präparate
Compositions cosmétiques fluorescentes

(30) Priority: 23.11.1988 US 275434
(43) Date of publication of application: 30.05.1990
(73) Proprietor: ESTEE LAUDER INC., New York New York 10153 (US)
(72) Inventor: Peters, David W., Amityville New York 11701 (US); Calvo, Luis C., Bayshore New York 11706 (US)
(74) Representative: VOSSIUS & PARTNER

(56) References cited:
- GB-A- 770 889
- GB-A- 820 111

## Description

This invention relates to colored cosmetic compositions. More particularly, this invention relates to cosmetic compositions that fluoresce when applied to a person's skin (e.g., the lips or cheeks), hair or nails, and exposed to incident light, and to a method for providing an attractive fluorescent appearance to the skin, hair or nails.

The use of cosmetics is widespread in modern society. Cosmetics typically are intended to provide an attractive appearance through the use of color, e.g., by highlighting certain features of the face and/or accentuating natural colors. Colored cosmetics can be used, for example, to accentuate lines of separation (lip liners), to provide sensuous color to portions of the skin (lipsticks and glosses) and to provide a "healthy glow" to the cheeks (blushes and rouges). Cosmetics may also be used to hide imperfections of the skin and to protect the skin (e.g., by blocking the skin from harmful ultraviolet light).

Dyes are known that fluoresce in the visible range in response to radiation in the ultraviolet and/or visible ranges. These dyes, which fluoresce when dissolved in a suitable solvent, but not in their pure, dry powder state, are referred to herein as "daylight fluorescent dyes". The dyes typically are dissolved in a carrier resin to obtain a solid solution, which then may be ground into a powder that exhibits fluorescent effects. Such powders are referred to herein as "daylight fluorescent pigments", the manufacture of which is described generally in US-A-2,851,424, 3,711,604, 3,856,550 and 2,938,878, which patents are incorporated herein by reference.

The daylight fluorescent pigments as cited above to date have not been utilized in cosmetic compositions. Neither have the daylight fluorescent pigments as cited above been applied to the skin (e.g., the lips or cheeks), hair, or nails to enhance their appearance.

GB-A-820 111 discloses a lipstick, a nail varnish or a nail polish comprising lakes of fluorescent dyes (such as discribed in GB-A-733 856) precipitated in the presence of synthetic resins, which when suspended in a finely powdered condition in a solid or liquid vehicle have the property of fluore cence in daylight.

It is an object of the present invention to provide cosmetic compositions comprising daylight fluorescent pigments that are characterized by exceptional brightness of color.

A further object of this invention is to provide attractive colored cosmetic compositions that are stable and safe for application to the human skin, hair or nails.

Another object of this invention is to provide a method of providing an attractive, fluorescent appearance to the skin, hair or nails.

The present invention relates to compositions and methods for achieving the foregoing objects. The compositions comprise:
(a) a daylight fluorescent pigment comprising a daylight fluorescent dye dissolved in a carrier resin, and
(b) a cosmetic carrier having admixed therein the daylight fluorescent pigment in an amount effective to provide a fluorescent appearance to the compositions when they are applied to a person's skin, hair or nails. The methods of this invention comprise applying an effective amount of the foregoing compositions to a person's skin (e.g., lips or cheeks), hair or nails.

The daylight fluorescent pigment component of the compositions of this invention should be present in an amount sufficient to provide the skin, hair, or nails with an aesthetically pleasing fluorescent appearance. Preferably the daylight fluorescent pigment comprises about 0.5-50% by weight of the cosmetic composition. The daylight fluorescent dye solution preferably comprises about 0.1-50% by weight of the daylight fluorescent pigment, on a dry weight basis. Generally, the more opaque the cosmetic carrier, the greater the amount of pigment and/or dye needed to achieve a desired effect.

The compositions of this invention may take a wide variety of forms, e.g., lipsticks, glosses, blush powders, mascaras and other similar make-up compositions. The cosmetic carrier may comprise a wide variety of ingredients that are conventionally used in cosmetics, e.g., waxes, mineral oils, fatty alcohols, glycerine, and sunscreens.

Any daylight fluorescent dye may be used in the compositions of this invention, provided it is safe for application to the skin, hair or nails, has a desirable color and is compatible with the other components of the composition. It is desirable to use a dye that is approved for drug and cosmetic use (D&C dyes) or food, drug and cosmetic use (FD&C dyes). The preferred fluorescent dyes for use in the compositions of this invention are FD&C Red #3, D&C Red #22, D&C Red #28, D&C Yellow #8, D&C Orange #5, D&C Orange #11 and D&C Green #8. Such dyes are well known, commercially available materials, with their chemical structure being described, e.g., in 21 C.F.R. Part 74 (as revised April 1, 1988) and the CTFA Cosmetic Ingredient Handbook, (1988), published by the Cosmetics, Toiletry and Fragrancy Association, Inc. These publications are incorporated herein by reference.

Any carrier resin may be used in the compositions of this invention provided it can be pulverized to a fine powder, is safe for application to the skin, and is compatible with the other components of the composition. The carrier resin may be thermoplastic or thermosetting. The use of thermosetting materials is generally preferred because such materials are more readily ground to minute pigment-size particles, without tending to "gum-up" or agglomerate during grinding.

Preferred carrier resins are those that do not absorb appreciable amounts of incident light, especially in the visible spectrum. Preferably, the carrier resins are transparent or at least translucent.

Polymeric materials approved by the Food and Drug Administration as "Indirect Food Additives" are especially preferred carrier resins for use in the make-up compositions of this invention. These materials, of the type listed in 21 C.F.R. Part 177 (as revised April 11, 1988), which publication is incorporated herein by reference, include, e.g., acrylic, cellophane, fluorocarbon, polyamide, polyester and polysulfone resins.

The daylight fluorescent pigments used in the compositions of this invention can be made by dissolving one or more daylight fluorescent dyes in the chosen carrier resin while the resin is in fluid or liquid form (e.g., during the manufacture of the resin, or by heating the already formed resin above its melting point). The daylight fluorescent pigments used in the compositions of this invention may also be made by contacting dry, powdered resin with one or more daylight fluorescent dyes that have been solubilized in an acid solution, and allowing the dye to be absorbed into the resin.

The resulting daylight fluorescent pigment is then allowed to harden by cooling or curing and, thereafter, is pulverized to the desired particle size. After pulverization, the average size of the daylight fluorescent pigment particles may be narrowed by any of various systems of classification, (e.g., by sieving or by air classification). Preferably, the pigment particles are substantially uniform in size and do not exceed about 100 or 150 microns in diameter. Very fine particle sizes in the range of about 5-25 microns are most satisfactory for use in the compositions of this invention, although larger and smaller particle sizes may be used.

Depending on the color effect desired, a mixture of fluorescent dyes may be used in the compositions of this invention. Suitable such mixtures include, for example, mixtures of (a) D&C Yellow #8 and D&C Red #28, (b) D&C Yellow #8 and D&C Red #22, (c) D&C Red #28 and D&C Red #22, and (d) D&C Orange #5 and D&C Red #28.

Also depending on the color effect desired, one or more non-fluorescent dyes or pigments may also be included in the compositions of this invention. Virtually all (if not all) of the coloring materials that are currently being used in commercial cosmetic compositions are suitable for such use. Among such materials are the lake of D&C Red #3, D&C Red #6, D&C Red #7, the lake of D&C Red #21, the lake of D&C Red #27, D&C Red #30, D&C Red #33, D&C Red #36, the lake of D&C Red #40, the lake of FD&C Yellow #6, D&C Yellow #6, the lake of D&C Yellow #10, the lake of FD&C Blue #1, and the lake of D&C Blue #1. These materials are listed in 21 C.F.R. Parts 74 and 82 (as revised April 1, 1988), which publication is incorporated herein by reference. Additionally, any of the colorants listed in Subparts A-C of 21 C.F.R. Part 73, (as revised April 1, 1988), which publication is incorporated herein by reference, may be included in the compositions of this invention.

While the compositions of this invention may be applied to any portion of the skin or hair, in the most preferred embodiments the compositions are applied to the cheeks or to the lips. The compositions, therefore, preferably takes a form suitable for such applications, e.g., the form of a lipstick or a powder for application to the cheeks.

The cosmetic carrier for the daylight fluorescent pigment or pigments is chosen to provide a composition that has the desired form (e.g., an emulsion, lipstick or powder). The cosmetic carrier should, of course, also be compatible with the daylight fluorescent pigment and be suitable for application to the skin, hair, or nails of a person.

Suitable cosmetic carriers are well known in the cosmetic art and include a vast array of materials. For example:
(1) when the composition takes the form of stick (e.g., a lipstick), at least a portion of the carrier typically will be a wax. Suitable waxes may be selected from the group consisting of lanolin, beeswax, candelilla wax, carnauba wax, cocoa butter, silicone waxes, fatty acids having a chain length of C12-C22, salts of the foregoing fatty acids, and mixtures thereof. In addition, the carrier may also include one or more oils, such as oils selected from the group consisting of paraffin oil, purcellin oil, sweet almond oil, avocado oil, castor oil, sesame oil, jojoba oil, mineral oils, silicone oils, cereal-germ oils, and mixtures thereof. The carrier may also include any number of colorants, flavorings or perfumes that are conventionally used in lipsticks.
(2) When the composition takes the form of a powder (e.g., a rouge composition for application to the cheeks), the carrier typically will comprise a mineral or organic filler, such as materials selected from the group consisting of talc, kaolin, starch, polyethylene powder, polyamide powder and mixtures thereof. The carrier may also include other materials that are conventionally used in many commercial cosmetic powders such as binders and colorants.
(3) When the composition takes the form of a semi-liquid, the carrier typically comprises a mixture of one or more waxes and one or more oils. The same waxes and oils that may be used to make the the lipsticks of this invention may also be used to make products of the invention that are in the form of semi-liquids, except that the specific oil and wax components and the amounts of those components are selected to provide a product that is in the form of a semi-liquid, as opposed to a stick, at room temperature.

Regardless of the form of the product, the compositions of this invention may also include one or more ingredients that are conventionally used in cosmetic compositions. Such ingredients include, for example, perfumes; sunscreens, such as paraaminobenzoic acid (PABA) and its derivatives; anti-oxidants, such as butylated hydroxyanisole, butylated hydroxytoluene, tocopherol and ascorbyl palmitate; emulsifying agents; and preservatives, such as butyl paraben and ethyl paraben.

In addition to protecting the skin from the harmful effects of ultraviolet light, sunscreens such as PABA perform the additional function of diminishing the harmful degradative effects of such light on the daylight fluorescent pigments, which can cause such pigments to fade over time. Other ultraviolet absorbers that are not conventionally classified as sunscreens, such as titanium dioxide, may also be included in the compositions of this invention for the purpose of diminishing the harmful degradative effects of ultraviolet light on the pigments and colorants in the compositions.

The pigments and cosmetic carriers may be combined to prepare the compositions of this invention by techniques conventionally used to prepare cosmetic compositions.

The following non-limiting examples illustrate the present invention.

### EXAMPLE I

This example illustrates a procedure for making a daylight fluorescent pigment for cosmetic use from the following ingredients: deionized water, sodium octoxynol-2 ethane sulfonate (TRITON X200, Rohm & Haas Co.), glacial acetic acid, D&C Yellow #8 dye, and powdered toluene sulfonamide formaldehyde resin (SANTOLITE, Monsanto Corp.).

### Procedure

3 g of TRITON X200 (a wetting agent that facilitates dye penetration) and 30 g of glacial acetic acid were dissolved into 120 g of deionized water. 0.3 g of D&C Yellow #8 were added to the solution, and mixed for 10 minutes using a suitable laboratory mixer at a low speed, e.g., 30 rpms. Then 15.0 g of the powdered toluene sulfonamide formaldehyde resin were added and the slurry was mixed for 3 additional minutes at the same speed.

The excess water was then decanted and the remaining pigment washed 8 times with deionized water. After the final decanting, the resulting fluorescent pigment was dried and pulverized using a mortar and pestle.

All the foregoing steps were carried out at room temperature.

### EXAMPLE II

This example illustrates a procedure for making a lipstick composition containing a fluorescent pigment, from the following ingredients: castor oil, candelilla, carnauba, castor wax, beeswax, ozokerite, lanolin, lanolin oil, mineral oil, butyl stearate, castor oil, and a fluorescent pigment (i.e., the Example I fluorescent pigment).

### Procedure

41.00 g of castor oil, 7.80 g of candelilla, 2.00 g of carnauba, 0.60 g of castor wax, 2.40 g of beeswax, 3.60 g of ozokerite, 7.80 g of lanolin, 7.80 g of lanolin oil, 3.00 g of mineral oil, and 9.00 g of butyl stearate were combined, heated to 80°C and mixed gently until homogeneous. 3.00 g of the Example I fluorescent pigment was then added to 12.00 g of castor oil and ground 3 times through a roller mill. This was added to the oil/wax mixture, which was then mixed until homogeneous, cooled to 60°C and poured into lipstick molds. The resulting sticks were cooled, withdrawn from the molds and fitted into lipstick cases.

### EXAMPLE III

This example illustrates a procedure for making a pressed powder blush containing a fluorescent pigment, from the following ingredients: talc, zinc stearate, mica, fluorescent pigment (i.e., the Example I fluorescent pigment), cetyl alcohol, mineral oil (70 cps), and octyl dodecanol (STANDAMUL G, Henkel).

### Procedure

66.0 g of talc, 2.0 g of zinc stearate, 6.0 g of mica and 20.0 g of the Example I fluorescent pigment were blended together and pulverized to yield a finely divided powder.

1.2 g of cetyl alcohol, 1.2 g of mineral oil and 3.6 g of octyl dodecanol were combined, heated to 65°C and mixed gently until homogenous. The resulting mixture was then sprayed over the above described powder. This was then blended until uniform in a ribbon blender.

## Claims

1. A cosmetic composition comprising :
(a) a daylight fluorescent pigment comprising a daylight fluorescent dye dissolved in a carrier resin, and (b) a cosmetic carrier having admixed therein said daylight fluorescent pigment in an amount effective to provide a fluorescent effect to the composition when it is applied to a person's skin, hair or nails.

2. The composition according to claim 1, wherein the daylight fluorescent dye is FD&C Red #3, D&C Yellow #8, D&C Red #22, D&C Red #28, D&C Orange #5, D&C Orange #11, D&C Green #8 or a mixture thereof.

3. The composition according to any one of claims 1 and 2 wherein the carrier resin is an acrylic, cellophane, fluorocarbon, polyamide, or polyester resin.

4. The composition according to claim 1 wherein the daylight fluorescent pigment contains about 0.1-50% by weight of the daylight fluorescent dye.

5. The composition according to claim 4 wherein the composition contains about 0.5-50% by weight of the daylight fluorescent pigment.

6. The composition according to claim 1, wherein the composition is in the form of a stick suitable for application to the lips.

7. The composition according to claim 1, wherein the composition is in the form of a powder.

8. The composition according to claim 1, wherein the composition is in the form of a semi-liquid.

9. A method for providing the skin, hair or nails of a person with an attractive fluorescent appearance comprising applying to the skin, hair or nails an effective amount of the composition of any one of claims 1 to 5 or 8.

10. A method for providing the lips of a person with an attractive fluorescent appearance comprising applying to the lips an effective amount of the composition of claim 6.

11. A method for providing the skin or hair of a person with an attractive fluorescent appearance comprising applying to the skin or hair an effective amount of the composition of claim 7.

12. The method of claim 11 wherein the composition is applied to the cheeks of a person.

## Patentansprüche

1. Kosmetisches Mittel, umfassend:
(a) ein Tageslichtfluoreszenzpigment umfassend einen Tageslichtfluoreszenzfarbstoff gelöst in einem Trägerharz und (b) einen kosmetischen Träger, in den das Tageslichtfluoreszenzpigment in einer Menge eingemischt ist, die ausreicht, dem Mittel einen Fluoreszenzeffekt zu verleihen, wenn es auf Haut, Haare oder Nägel einer Person aufgebracht wird.

2. Mittel nach Anspruch 1, wobei der Tageslichtfluoreszenzfarbstoff FD&C Red #3, D&C Yellow #8, D&C Red #22, D&C Red #28, D&C Orange #5, D&C Orange #11, D&C Green #8 oder ein Gemisch davon ist.

3. Mittel nach einem der Ansprüche 1 und 2, wobei das Trägerharz ein Acryl-, Cellophan-, Fluorkohlenstoff-, Polyamid- oder Polyesterharz ist.

4. Mittel nach Anspruch 1, wobei das Tageslichtfluoreszenzpigment etwa 0,1 bis 50 Gew.-% Tageslichtfluoreszenzfarbstoff enthält.

5. Mittel nach Anspruch 4, wobei das Mittel etwa 0,5 bis 50 Gew.-% Tageslichtfluoreszenzpigment enthält.

6. Mittel nach Anspruch 1, wobei das Mittel in Form eines Stiftes vorliegt, der für die Anwendung auf die Lippen geeignet ist.

7. Mittel nach Anspruch 1, wobei das Mittel in Form eines Pulvers vorliegt.

8. Mittel nach Anspruch 1, wobei das Mittel in Form einer Semiflüssigkeit vorliegt.

9. Verfahren zur Ausstattung der Haut, Haare oder Nägel einer Person mit einem attraktiven fluoreszierenden Aussehen umfassend das Aufbringen einer wirksamen Menge des Mittels nach einem der Ansprüche 1 bis 5 oder 8 auf die Haut, Haare oder Nägel.

10. Verfahren zur Ausstattung der Lippen einer Person mit einem attraktiven fluoreszierenden Aussehen umfassend das Aufbringen einer wirksamen Menge des Mittels nach Anspruch 6 auf die Lippen.

11. Verfahren zur Ausstattung der Haut oder Haare einer Person mit einem attraktiven fluoreszierenden Aussehen umfassend das Aufbringen einer wirksamen Menge des Mittels nach Anspruch 7 auf die Haut oder Haare.

12. Verfahren nach Anspruch 11, wobei das Mittel auf die Wangen einer Person aufgebracht wird.

## Revendications

1. Composition cosmétique contenant :
a) un pigment fluorescent à la lumière du jour contenant un colorant fluorescent à la lumière du jour dissout dans une résine support, et
b) un support cosmétique qui a, mélangé dans celui-ci, ce pigment fluorescent à la lumière du jour en quantité efficace pour fournir un effet de fluorescence à la composition lorsqu'elle est appliquée sur la peau d'une personne, sur les cheveux ou sur les ongles.

2. Composition selon la revendication 1, caractérisée en ce que le colorant fluorescent à la lumière du jour est F. D. et C. Red # 3, D. et C. Yellow # 8, D. et C. Red # 22 ; D. et C. # 28, D. et C. Orange # 5, D. et C. Orange # 11, D. et C. Green # 8 ou un mélange de ceux-ci.

3. Composition selon l'une des revendications 1 et 2, dans laquelle la résine support est une résine acrylique, de la cellophane, un fluorocarbure, un polyamide, ou une résine de polyester.

4. Composition selon la revendication 1, caractérisée en ce que le pigment fluorescent à la lumière du jour contient environ 0,1 à 50 % en poids de colorant fluorescent à la lumière du jour.

5. Composition selon la revendication 4, caractérisée en ce que la composition contient environ 0,5 à 50 % en poids de pigment fluorescent à la lumière du jour.

6. Composition selon la revendication 1, caractérisée en ce que la composition est sous la forme d'un bâton approprié pour l'application sur les lèvres.

7. Composition selon la revendication 1, caractérisée en ce que la composition est sous la forme d'une poudre.

8. Composition selon la revendication 1, caractérisée en ce que la composition est sous la forme d'un produit semi-solide.

9. Une méthode pour fournir à la peau, aux cheveux et aux ongles d'une personne, un aspect attirant, fluorescent, qui comprend le fait d'appliquer sur la peau, les cheveux ou les ongles une quantité efficace de la composition d'une quelconque des revendications 1 à 5 et 8.

10. Une méthode pour fournir aux lèvres d'une personne un aspect fluorescent attirent, qui comprend le fait d'appliquer sur les lèvres une quantité efficace de la composition selon la revendication 6.

11. Une méthode pour fournir à la peau, ou aux cheveux d'une personne un aspect fluorescent attirant qui comprend le fait d'appliquer sur la peau ou sur les cheveux une quantité efficace de la composition selon la revendication 7.

12. Méthode selon la revendication 11, caractérisée en ce que la composition est appliquée sur les joues d'une personne.
